(19) Europäisches Patentamt
European Patent Office
Office européen des brevets

(11) Veröffentlichungsnummer: **0 132 508**
**A2**

(12) # EUROPÄISCHE PATENTANMELDUNG

(21) Anmeldenummer: **84104550.3**

(22) Anmeldetag: **21.04.84**

(51) Int. Cl.⁴: **C 07 D 233/60**
C 07 D 249/08, C 07 D 403/06
A 61 K 31/41, A 61 K 31/415

(30) Priorität: **30.04.83 DE 3315808**

(43) Veröffentlichungstag der Anmeldung:
**13.02.85 Patentblatt 85/7**

(84) Benannte Vertragsstaaten:
**BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Paessens, Arnold, Dr.**
**Stresemannstrasse 56**
**D-5657 Haan 1(DE)**

(72) Erfinder: **Streissle, Gert, Dr.**
**Am Hochsitz 17**
**D-5600 Wuppertal(DE)**

(72) Erfinder: **Plempel, Manfred, Dr.**
**Zwengenbergerstrasse 3c**
**D-5657 Haan(DE)**

(72) Erfinder: **Büchel, Karl Heinz, Prof. Dr.**
**Dabringhausener Strasse 42**
**D-5093 Burscheid(DE)**

(72) Erfinder: **Elbe, Hans-Ludwig, Dr.**
**Dasnöckel 59**
**D-5600 Wuppertal 11(DE)**

(72) Erfinder: **Holmwood, Graham, Dr.**
**Krutscheider Weg 105**
**D-5600 Wuppertal 11(DE)**

(72) Erfinder: **Kraatz, Udo, Dr.**
**Andreasstrasse 22a**
**D-5090 Leverkusen(DE)**

(72) Erfinder: **Regel, Erik, Dipl.-Ing.**
**Untere Bergerheide 26**
**D-5600 Wuppertal(DE)**

(54) **Hydroxyethyl-Azolyl-Derivate, ihre Verwendung und diese enthaltende Antiviralmittel.**

(57) Hydroxyethyl-azolyl-derivate der allgemeinen Formel

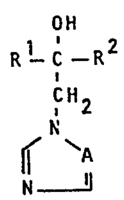

in der A, R¹ und R² die in der Beschreibung angegebene Bedeutung haben, sind stark wirksam gegen Viren und sollen als Wirkstoffe in antiviralen Mitteln Verwendung finden.

EP 0 132 508 A2

BAYER AKTIENGESELLSCHAFT          5090 Leverkusen, Bayerwerk
Konzernverwaltung RP
Patentabteilung                   Si/ABc

BEZEICHNUNG GEÄNDERT
Siehe Titelseite

Antivirale Mittel

Die vorliegende Erfindung betrifft antivirale Mittel, die Hydroxyethyl-azolyl-Derivate als Wirkstoffe enthalten.

Es wurde gefunden, daß die Hydroxyethyl-azolyl-Derivate der Formel

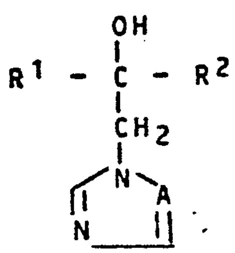

(I)

in welcher

A für ein Stickstoffatom oder die CH-Gruppe steht,

$R^1$ für jeweils gegebenenfalls im Phenylteil substituiertes Phenyl, Phenoxymethyl, Phenethyl oder Phenethenyl steht, sowie für Naphthyloxymethyl, 1,2,4-Triazol-1-yl-methyl oder Imidazol-1-yl-methyl steht,

$R^2$ für die Gruppierung $-C(CH_3)_2-R^3$, 1,2,4-Triazol-1-yl-methyl oder Imidazol-1-yl-methyl steht und

$R^3$ für Methyl sowie jeweils gegebenenfalls substituiertes Phenoxy oder Benzyl steht,

mit der Maßgabe, daß nicht gleichzeitig A für ein Stickstoffatom, $R^1$ für p-Chlorphenoxymethyl und $R^2$ für tert.-Butyl stehen,

und deren physiologisch verträglichen Säureadditions-Salze starke antivirale Wirkungen aufweisen.

Überraschenderweise zeigen die Hydroxyethyl-azolyl-Derivate der Formel (I) eine bessere antivirale Wirksamkeit als aus dem Stand der Technik bekannte antiviral wirksame Verbindungen.

Die erfindungsgemäß zu verwendenden Hydroxyethyl-azolyl-Derivate sind durch die Formel (I) allgemein definiert. In dieser Formel stehen vorzugsweise

A für die in der Erfindungsdefinition angegebene Bedeutung;

$R^1$ für jeweils gegebenenfalls einfach bis dreifach, gleich oder verschieden im Phenylteil substituiertes Phenyl, Phenoxymethyl, Phenethyl, oder Phenethenyl, wobei als Substituenten genannt seien: Halogen, Alkyl mit 1 bis 4 Kohlenstoffatomen, Alkoxy und Alkylthio mit jeweils 1 bis 2 Kohlenstoffatomen, Halogenalkyl und Halogenalkoxy sowie Halogenalkylthio mit jeweils 1 bis 2 Kohlenstoffatomen mit 1 bis 5 gleichen oder verschiedenen Halogenatomen, wie Fluor- und Chloratomen, Phenyl und Halogenphenyl; ferner für Naphthyloxymethyl, 1,2,4-Triazol-1-yl-methyl und Imidazol-1-yl-methyl;

$R^2$ für die Gruppierung $-C(CH_3)_2-R^3$, für 1,2,4-Triazol-1-yl-methyl oder Imidazol-1-yl-methyl; und

$R^3$ für Methyl sowie für jeweils gegebenenfalls einfach bis dreifach, gleich oder verschieden substituiertes Phenoxy oder Benzyl, wobei als Substituenten vorzugsweise die bei $R^1$ bereits vorzugsweise genannten Phenylsubstituenten in Frage kommen;

mit der Maßgabe, daß nicht gleichzeitig A für ein Stickstoffatom, $R^1$ für p-Chlorphenoxymethyl und $R^2$ für tert.-Butyl stehen.

Besonders bevorzugt sind diejenigen Verbindungen der Formel (I), in denen

A) $R^1$ für jeweils gegebenenfalls einfach bis dreifach, gleich oder verschieden im Phenylteil substituiertes Phenoxymethyl, Phenethyl oder Phenethenyl steht, wobei als Substituenten beispielsweise genannt seien: Fluor, Chlor, Methyl, Trifluormethyl, Trifluormethoxy, Trifluormethylthio, Phenyl und Chlorphenyl; sowie für Naphthyloxymethyl steht; und

$R^2$ für tert.-Butyl steht, wobei nicht gleichzeitig A für ein Stickstoffatom und $R^1$ für p-Chlorphenoxy stehen;

B) $R^1$ für gegebenenfalls einfach bis zweifach, gleich oder verschieden im Phenylteil substituiertes Phenoxymethyl steht, wobei als Substituenten die unter Punkt A bereits genannten Phenylsubstituenten in Frage kommen; und

$R^2$ für die Gruppierung $-C(CH_3)_2-R^3$ steht, wobei

$R^3$ für gegebenenfalls einfach bis zweifach, gleich oder verschieden substituiertes Phenoxy steht, wobei als Substituenten die unter Punkt A für $R^1$ bereits genannten Phenylsubstituenten in Frage kommen;

C) $R^1$ für gegebenenfalls einfach bis zweifach, gleich oder verschieden substituiertes Phenyl steht, wobei als Substituenten die unter Punkt A bereits genannten Phenylsubstituenten in Frage kommen; und

$R^2$ für die Gruppierung $-C(CH_3)_2-R^3$ steht, wobei

$R^3$ für gegebenenfalls einfach bis zweifach, gleich oder verschieden substituiertes Phenoxy steht, wobei als Substituenten die unter Punkt A für $R^1$ bereits genannten Phenylsubstituenten in Frage kommen;

D) $R^1$ für gegebenenfalls einfach bis zweifach, gleich oder verschieden im Phenylteil substituiertes Phenoxymethyl steht, wobei als Substituienten die unter Punkt A bereits genannten Phenylsubstituenten in Frage kommen; und

$R^2$ für 1,2,4-Triazol-1-yl-methyl oder Imidazol-1-ylmethyl steht;

E) $R^1$ für 1,2,4-Triazol-1-yl-methyl oder Imidazol-1-yl-methyl steht; und

$R^2$ für die Gruppierung $-C(CH_3)_2-R^3$ steht, wobei

$R^3$ für gegebenenfalls einfach bis zweifach, gleich oder verschieden substituiertes Benzyl steht, wobei als Phenylsubstituenten die unter Punkt A für $R^1$ bereits genannten Phenylsubstituenten in Frage kommen.

Bevorzugte erfindungsgemäße Verbindungen sind auch Additionsprodukte aus Säuren und denjenigen Hydroxyethyl-azolyl-Derivaten der Formel (I), in denen die Substituenten A, $R^1$ und $R^2$ die Bedeutungen haben, die bereits vorzugsweise für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z.B. p-Toluolsulfonsäure und 1,5-Naphthalindisulfonsäure.

Die erfindungsgemäß zu verwendenden Wirkstoffe sind bekannt (vgl. EP 0 040 345 und EP 0 043 419 sowie DE-OS 32 24 186), bzw. sind sie Gegenstand eigener, älterer Patentanmeldungen, die noch nicht veröffentlicht sind (vgl. die deutschen Patentanmeldungen P 32 02 613, P 32 02 601, P 32 12 388,

P 32 32 647 und P 33 07 217) und können nach den dort angegebenen Verfahren erhalten werden, indem man z.B. Oxirane der Formel

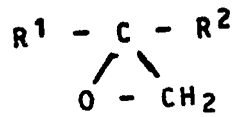

(II)

in welcher

$R^1$ und $R^2$ die oben angegebene Bedeutung haben,

mit Azolen der Formel

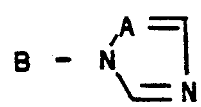

(III)

in welcher

A die oben angegebene Bedeutung hat und
B für Wasserstoff oder ein Alkalimetall, wie Natrium oder Kalium steht,

in Gegenwart eines inerten organischen Lösungsmittels, wie beispielsweise Alkohole, und gegebenenfalls in Gegenwart einer Base, wie beispielsweise Natriumalkoholat, gegebenenfalls unter einem Druck von 1 bis 25 bar, bei Temperaturen zwischen 60 und 150°C umsetzt.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäßen Verbindungen der Formel (I) weisen wie bereits erwähnt, starke antivirale Wirkungen auf. Diese Wirkungen sind besonders ausgeprägt bei lipidhaltigen Viren, wie z.B. Herpes-Viren, und auch bei nichtlipidhaltigen Viren, wie z.B. Papillom-Viren.

Als Indikationen in der Humanmedizin können für Herpes-Virusinfektionen beispielsweise genannt werden:

Herpes labialis, Herpes genitalis, Keratoconjunctivitis herpetica, Varizellen (Windpocken), Herpes zoster (Gürtelrose), Mononucleose sowie Infektionen mit Cytomegalie-Virus.

Als Indikationen in der Tiermedizin können für Herpes-Virusinfektionen aufgeführt werden:

Infektionen mit Pseudorabies-Virus (Rind, Schwein), Rhinotracheitis-Viren (Rind), Rhinopneumonitis-Virus (Pferd) sowie Marek Virus (Huhn).

Als Indikationen in der Human- und Tiermedizin seien für Papillom-Virusinfektionen Virus-bedingte Warzen bekannt.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1,2,3 oder 4 Einzeldosen oder 1/2, 1/3 oder 1/4 eine Einzeldosis enthalten. Eine Einzeldosis enthält vorzugsweise die Mengen Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glucose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Den Tabletten, Dragees, Kapseln, Pillen und Granulaten können mit den üblichen gegebenenfalls Opakisierungsmittel enthaltenen Überzügen und Hüllen versehen sein und so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben gangegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffen enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsverzögerer und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylalkohol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen dem oder der Wirkstoffe mit dem oder den Trägerstoffen.

Zur vorliegenden Erfindung gehört auch die Verwendung der erfindungsgemäßen Wirkstoffe, sowie von pharmazeutischen Zubereitungen, die einen oder mehrere erfindungsgemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rektal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 2,5 bis etwa 200, vorzugsweise 5 bis 150 mg/kg Körpergewicht je 24 Stunden, gegebenenfalls in Form mehrerer Einzelgaben zur Erzielung der gewünschten Ergebnisse zu verabreichen.

Bei oraler Applikation werden die erfindungsgemäßen Wirkstoffe in Gesamtmengen von etwa 2,5 bis etwa 200, vorzugsweise 5 bis 150 mg/kg Körpergewicht je 24 Stunden und bei parenteraler Applikation in Gesamtmengen von etwa 2,5 bis etwa 50, vorzugsweise 1 bis 25 mg/kg Körpergewicht je 24 Stunden verabreicht.

Es kann jedoch erforderlich sein, von den genannten Dosierungen abzuweichen und zwar in Abhängigkeit von der Art und dem Körpergewicht des zu behandelnden Objektes,

der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Herstellungsbeispiele

Beispiel (Ia-1)

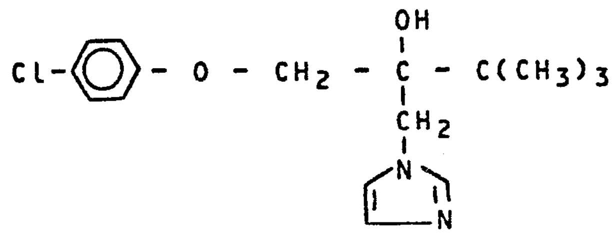

2,71 g (0,1178 Mol) Natrium in 250 ml absolutem Ethanol werden mit 8,02 g (0,1178 Mol) Imidazol versetzt. Innerhalb von 30 Minuten läßt man bei Raumtemperatur eine Lösung von 14,µ7 g (0,0589 Mol) 2-(4-Chlorphenoxy-methyl)-2-tert.-butyl-oxiran in 100 ml Ethanol zutropfen. Danach wird das Reaktionsgemisch 8 Stunden unter Rückfluß erhitzt, eingeengt und der Rückstand in Ether aufgenommen. Man extrahiert dreimal mit 1 n Salzsäure, neutralisiert die vereinigten Salzsäurephasen mit Natriumhydrogencarbonat und extrahiert anschließend mit Essigester. Nach dem Einengen und Umkristallisieren aus Cyclohexan erhält man 11,6 g (64 % der Theorie) 2-(4-Chlorphenoxy-methyl)-3,3-dimethyl-1-(imidazol-1-yl) butan-2-ol vom Schmelzpunkt 154-155°C.

Herstellung des Ausgangsproduktes

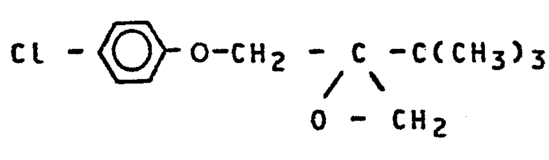

Eine Lösung von 189 ml (2,0 Mol) Dimethylsulfat in 1200 ml absolutem Acetonitril wird bei Raumtemperatur mit einer Lösung von 162 ml (2,2 Mol) Dimethylsulfid in 400 ml absolutem Acetonitril versetzt. Man läßt die Reaktionsmischung über Nacht bei Raumtemperatur rühren. Danach werden 118,8 g (2,2 Mol) Natriummethylat zugegeben. Man läßt 30 Minuten rühren und versetzt dann innerhalb von 30 Minuten tropfenweise mit einer Lösung von 272 g (1,2 Mol) 1-(4-Chlorphenoxy)-3,3-dimethylbutan-2-on in 600 ml absolutem Acetonitril. Man läßt das Reaktionsgemisch über Nacht nachrühren. Anschließend wird eingeengt, der Rückstand zwischen Wasser und Essigester verteilt, die organische Phase abgetrennt, zweimal mit Wasser und einmal mit gesättigter Natriumchloridlösung gewaschen, über Natriumsulfat getrocknet, eingeengt und der Rückstand im Vakuum destilliert. Man erhält 242,2 g (84 % der Theorie) 2-(4-Chlorphenoxymethyl)-2-tert.-butyl-oxiran vom Siedepunkt 115-22°C/ 0,003 mm Hg-Säule und vom Schmelzpunkt 50-52°C.

<u>Beispiel (Ia-2)</u>

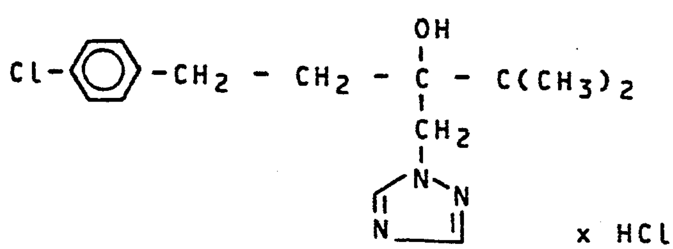

Eine Lösung von 17,9 g (0,075 Mol) 2-(4-Chlorphenylethyl)-2-tert.-butyl-oxiran und 6,9 g (0,1 Mol) 1,2,4-Triazol in 30 ml Ethanol wird 20 Stunden bei 150°C im Bombenrohr erhitzt. Man läßt abkühlen und engt die Reaktionslösung ein. Der Rückstand wird in Ether gelöst, dreimal mit Wasser und einmal mit Natriumchloridlösung gewaschen, über Natriumsulfat getrocknet und eingeengt. Der Rückstand wird über eine Kieselgelsäule chromatographiert (Laufmittel: Dichlorethan/Essigester 1:1). Man erhält 12,3 g (53,2 % der Theorie) 1-(4-Chlorphenyl)-4,4-dimethyl-3-(1,2,4-triazol-1-yl-methyl)-pentan-3-ol als zähes Öl.

Durch übliche Umsetzung mit etherischem Chlorwasserstoff erhält man nahezu quantitativ das Hydrochlorid vom Schmelzpunkt 212°C.

In entsprechender Weise werden die nachfolgenden Verbindungen der allgemeinen Formel (Ia)

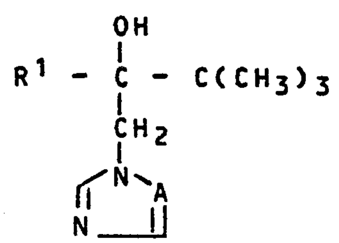

(Ia)

erhalten:

| Bsp.Nr. | $R^1$ | A | Schmelz-punkt |
|---|---|---|---|
| Ia-3 | 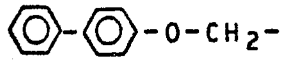 | CH | 169-70,5 |
| Ia-4 | 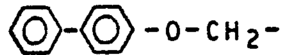 | N | 118-19,5 |
| Ia-5 | 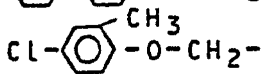 | N | 125,5-29 |
| Ia-6 | 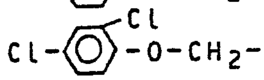 | N | 120,5-23,5 |
| Ia-7 | 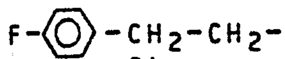 | N | 91-95,5 |
| Ia-8 | 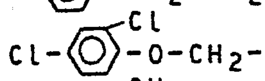 | CH | 152-54 |
| Ia-9 | 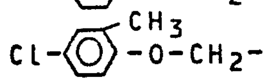 | CH | 157-59 |
| Ia-10 | 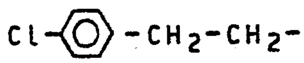 | CH | 157,5-59,5 |
| Ia-11 | 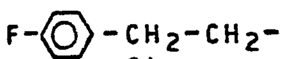 | CH | 124-25 |
| Ia-12 | 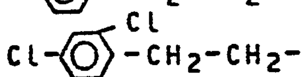 | CH | 118-19 |
| Ia-13 | 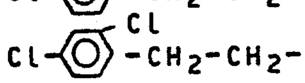 | N | 94-95 |
| Ia-14 | 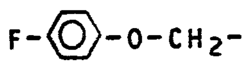 | CH | 141-42 |
| Ia-15 | 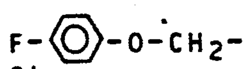 | N | 73-75 |
| Ia-16 | 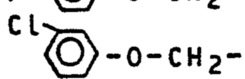 | CH | 124 |
| Ia-17 | 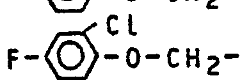 | CH | 137 |
| Ia-18 | 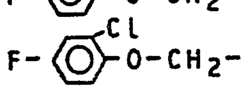 | N | 130 |
| Ia-19 | 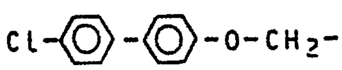 | CH | 174-76 |
| Ia-20 | 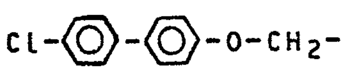 | N | 109-11 |
| Ia-21 | 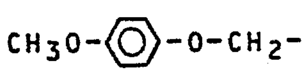 | N | 63-66 |
| Ia-22 | 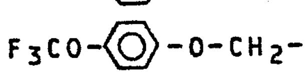 | N | $n_D^{20}$ =1,4902 |
| Ia-23 | 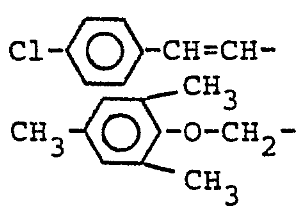 | N | 115-117 |
| Ia-24 | | N | 75-76,5 |

| Bsp. Nr. | $R^1$ | A | Schmelzpunkt |
|---|---|---|---|
| Ia-25 | 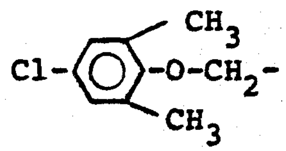 | N | 102-103,5 |
| Ia-26 | 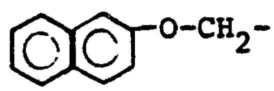 | N | 128,5-131 |

Entsprechend den Beispielen Ia-1 und Ia-2 werden die nachfolgenden Verbindungen der allgemeinen Formel (Ib)

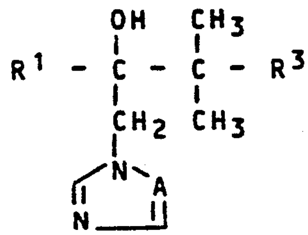

(Ib)

erhalten:

| Bsp.-Nr | $R^1$ | $R^3$ | A | Schmelzpunkt (°C) bzw. $n_D^{20}$ |
|---|---|---|---|---|
| Ib-1 | 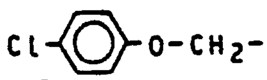 | 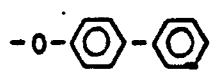 | N | Harz |
| Ib-2 | 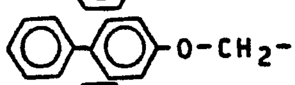 | 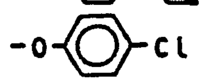 | N | Harz |
| Ib-3 | 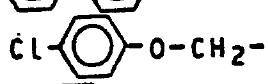 | 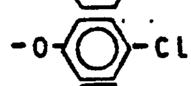 | N | Harz |
| Ib-4 | 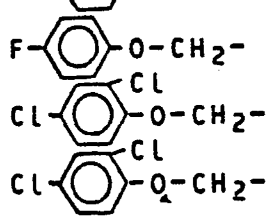 | 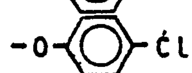 | N | Harz |
| Ib-5 | | 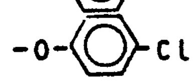 | N | Harz |
| Ib-6 | | 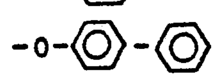 | N | Harz |

Beispiel Ic-1

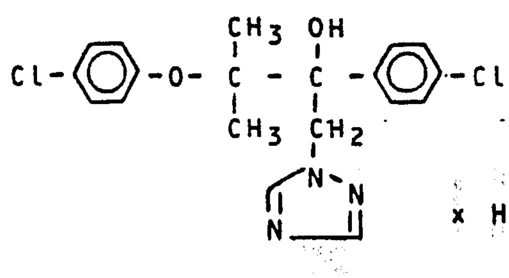

x HCl

Eine Lösung von 30 g (0,093 Mol) 2-(4-Chlorphenyl)-2-/2-(p-chlorphenoxy)-prop-2-yl7-oxiran in 40 ml n-Propanol wird bei Raumtemperatur zu einer Lösung von 7,6 g (0,107 Mol) 1,2,4-Triazol-Natrium in 60 ml n-Propanol getropft. Man läßt das Reaktionsgemisch 48 Stunden bei Rückflußtemperatur nachrühren, kühlt ab, versetzt mit Wasser und extrahiert mit Methylenchlorid. Die organische Phase wird über Natriumsulfat getrocknet und im Vakuum eingeengt. Der ölige Rückstand wird säulenchromatographisch gereinigt. Man erhält 6,7 g (18,4 % der Theorie) 3-(4-Chlorphenoxy)-2-(4-chlorphenyl)-3-methyl-1-(1,2,4-triazol-1-yl)-2-butanol. Diese werden bei Raumtemperatur mit 20 ml gesättigter Chlorwasserstoff/Ether-Lösung verrührt. Der ausfallende Niederschlag wird abgesaugt, mit wenig Ether nachgewaschen und im Vakuum bei 40°C getrocknet. Man erhält 6,5 g (89 % der Theorie bezogen auf eingesetzte Base) 3-(4-Chlorphenoxy)-2-(4-chlorphenyl)-3-methyl-1-(1,2,4-triazol-1-yl)-2-butanol-hydrochlorid vom Schmelzpunkt 135°C.

Herstellung des Ausgangsproduktes

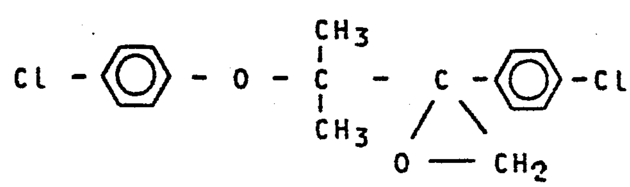

Eine Lösung von 59,2 g (0,47 Mol) Dimethylsulfat und 32 g (0,517 Mol) Dimethylsulfid in 270 ml Acetonitril

läßt man 5 Tage bei Raumtemperatur rühren. Innerhalb von ca. 2 Stunden läßt man dann bei 20 bis 25°C eine Lösung von 87 g 4-Chlorphenyl-/2-(p-chlorphenoxy)-prop-2-yl7-keton in 80 ml Acetonitril zugetropft. Bei gleicher Temperatur trägt man 28,7 g (0,53 Mol) Natriummethylat ein, läßt 12 Stunden nachrühren und engt anschließend ein. Der Rückstand wird mit einem Gemisch aus 200 ml Essigester und 150 ml Wasser über Nach verrührt. Die organische Phase wird abgetrennt, über Natriumsulfat getrocknet und im Vakuum eingeengt. Man erhält 49 g (76 % der Theorie) rohes 2-(4-Chlorphenyl)-2-/2-(p-chlorphenoxy)-prop-2-yl7-oxiran, das direkt weiter umgesetzt wird.

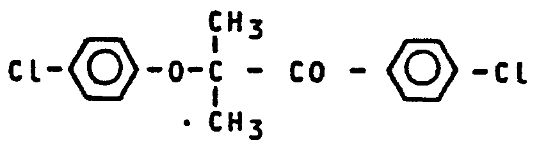

52 g (0,3982 Mol) p-Chlorphenol und 55 g (0,3982 Mol) Kaliumcarbonat in 400 ml Toluol werden 2 Stunden unter Rückfluß am Wasserabscheider erhitzt. Man kühlt auf 40°C ab und versetzt tropfenweise mit einer Lösung von 2-Brom-prop-2-yl-(4-chlorphenyl)-keton in 170 ml Toluol.

Dieses Reaktionsgemisch läßt man 5 Stunden bei 100°C nachrühren, kühlt anschließend ab, versetzt mit Wasser und trennt die organische Phase ab. Diese wird mit verdünnter Natronlauge sowie Wasser gewaschen, über Natriumsulfat getrocknet und eingeengt. Man erhält 87 g

(85 % der Theorie) rohes 4-Chlorphenyl-/2-(p-chlorphenoxy)-prop-2-yl/-keton, das direkt weiter umgesetzt wird.

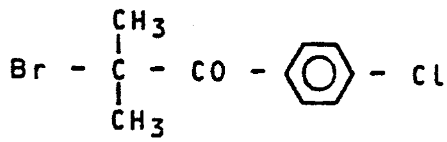

Zu 65,5 g (0,36 Mol) 4-Chlorphenyl-isopropyl-keton in 200 ml Chlorform gibt man 1 ml Bromwasserstoff/Eisessig zu und läßt dann bei 30°C 57,5 g (0,36 Mol) Brom zutropfen. Man läßt 30 Minuten bei Raumtemperatur nachrühren und engt danach im Vakuum ein. Man erhält 86,6 g (92 % der Theorie) rohes 2-Brom-prop-2-yl-(4-chlorphenyl)-keton, das direkt weiter umgesetzt wird.

In entsprechender Weise werden die nachfolgenden Verbindungen der allgemeinen Formel (Ic)

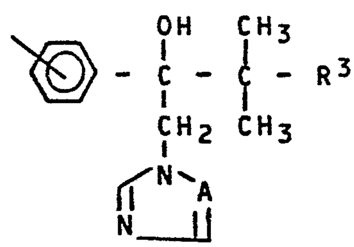

(Ic)

enthalten:

| Bsp.Nr. | 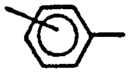 | $R^3$ | A | Schmelz-punkt (°C) |
|---|---|---|---|---|
| Ic-2 | 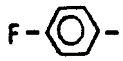 | 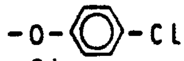 | N | 82 |
| Ic-3 | 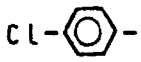 | 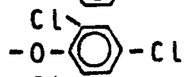 | CH | 80-82 |
| Ic-4 | 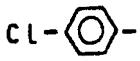 | 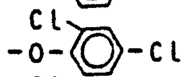 | N | 96-98 |
| Ic-5 | 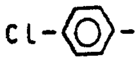 | 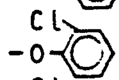 | N | 114-16 |
| Ic-6 | 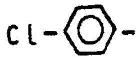 | 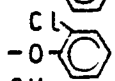 | CH | 194 |
| Ic-7 | 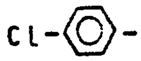 | 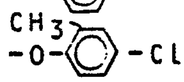 | N | 117 |
| Ic-8 | 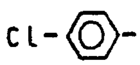 | 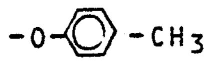 | N | 62 |
| Ic-9 | 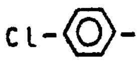 | 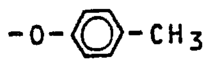 | CH | 106 |
| Ic-10 | 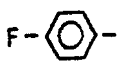 | 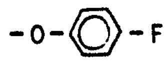 | N | 78 |
| Ic-11 | 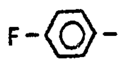 | 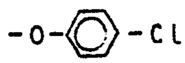 | CH | 154 |
| Ic-12 | 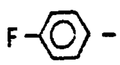 | 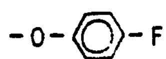 | CH | 178 |
| Ic-13 | 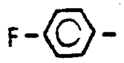 | 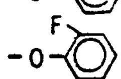 | N | 120 |
| Ic-14 | 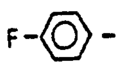 | 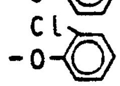 | N | 147 |

Beispiel (Id-1)

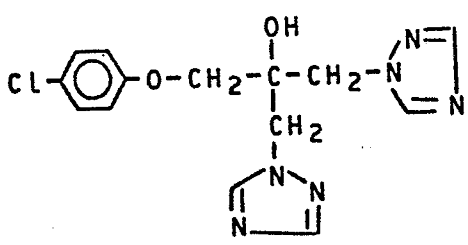

Zu einem Gemisch von 13,6 g (0,2 Mol) 1,2,4-Triazol und 13,8 g (0,1 Mol) Kaliumcarbonat in 200 ml Aceton werden unter Rühren 9,4 g (0,04 Mol) 2-Chlormethyl-2-(4-chlorphenoxymethyl)-oxiran getropft. Man läßt 15 Stunden bei Raumtemperatur und anschließend 22 Stunden unter Rückfluß rühren. Das Reaktionsgemisch wird danach kalt filtriert und das Filtrat im Vakuum eingeengt. Der ölige Rückstand wird in Chloroform gelöst, mit Wasser gewaschen, über Natriumsulfat getrocknet und chromatographisch (Kieselgel 60 Merck, Chloroform/Methanol = 20/1) gereinigt. Man erhält 5,8 g (43 % der Theorie) 2-(4-Chlorphenoxymethyl)-1,3-di-(1,2,4-triazol-1-yl)-2-hydroxypropan vom Schmelzpunkt 99°C.

Herstellung des Ausgangsproduktes

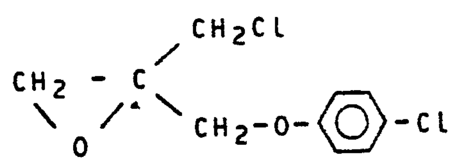

12,85 g (0,1 Mol) 4-Chlorphenol in 50 ml Aceton werden zu einem Gemisch von 14,1 g (0,1 Mol) 2,2-Di(chlormethyl)-oxiran, 13,8 g (0,1 Mol) Kaliumcarbonat in 200 ml Aceton getropft. Man erhitzt 18 Stunden unter Rückfluß, läßt abkühlen und filtriert. Das Filtrat wird im Vakuum eingeengt, der Rückstand in Chloroform gelöst, mit Wasser gewaschen, über Natriumsulfat getrocknet und eingeengt. Nach destillativer Reinigung erhält man 7,6 g (32,5 % der Theorie) 2-Chlormethyl-2-(4-chlorphenoxymethyl)-oxiran vom Siedepunkt 150°C/0,5 mbar.

In entsprechender Weise können die folgenden Verbindungen der allgemeinen Formel (Id)

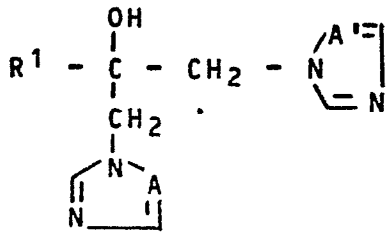

(Id)

erhalten werden:

| Bsp.Nr. | $R^1$ | A | A' | Schmelzpunkt(°C) |
|---|---|---|---|---|
| Id-2 | 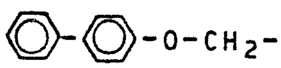 | N | N | 140 |
| Id-3 | 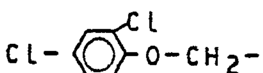 | N | N | 138 |
| Id-4 | 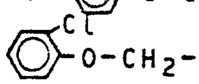 | N | N | 95 |
| Id-5 | 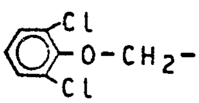 | N | N | 166 |
| Id-6 | 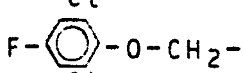 | N | N | 90 |
| Id-7 | 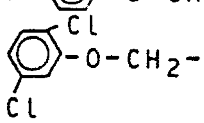 | N | N | 130 |
| Id-8 | 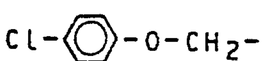 | CH | CH | 100 |
| Id-9 | 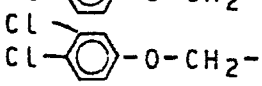 | N | N | 158 |
| Id-10 | 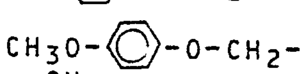 | N | N | 118 |
| Id-11 | 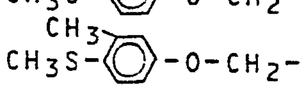 | N | N | 70 |
| Id-12 | 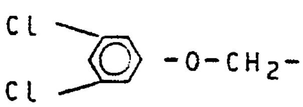 | N | N | 162 |
| Id-13 | 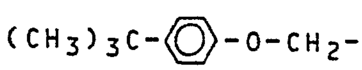 | N | N | 98 |
| Id-14 | 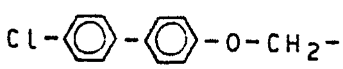 | N | N | 166 |

Beispiel (Ie-1)

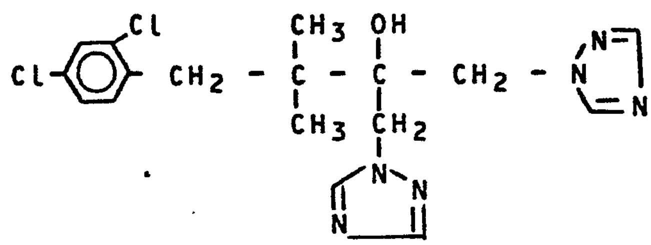

Zu einer Lösung von 0,11 g (47 mMol) Natrium in 30 ml n-Propanol werden bei Raumtemperatur unter Rühren 3,7 g (52,6 mMol) 1,2,4-Triazol gegeben. Man erhitzt auf Rückflußtemperatur und versetzt mit einer Lösung von 15,4 g (47 mMol) 2-(2,4-Dichlorphenyl-tert.-butyl)-2-(1,2,4-triazol-1-yl-methyl)-oxiran in 20 ml n-Propanol. Das Reaktionsgemisch wird 15 Stunden unter Rückfluß erhitzt, danach abgekühlt und auf Wasser gegeben. Man extrahiert mit Methylenchlorid, trocknet die organische Phase über Natriumsulfat und engt ein. Der Rückstand wird säulenchromatographisch (Kieselgel; Essigester: Cyclohexan = 3:1) gereinigt. Man erhält 3,5 g (18,8 % der Theorie) 4-(2,4-Dichlorphenyl)3,3-dimethyl-2-(1,2,4-triazol-1-yl-methyl)-1-(1,2,4-triazol-1-yl)-2-butanol vom Schmelzpunkt 126°C.

Herstellung des Ausgangsproduktes

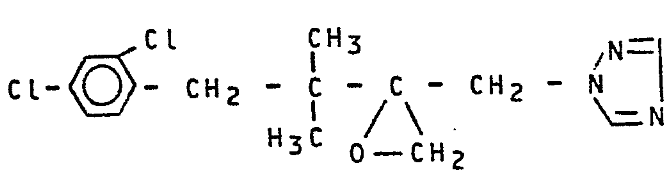

15,7 g (71,2 mMol) Trimethylsulfoniumiodid werden unter Stickstoffatmosphäre in 16 g Dimethylsulfoxid gelöst. Unter Kühlung wird bei Raumtemperatur mit 9,4 g (71,2 mMol) Kalium-tert.-butylat versetzt. Man läßt 6 Stunden nachrühren und versetzt anschließend mit einer Lösung von 20 g (64,1 mMol) 4-(2,4-Dichlorphenyl)-3,3-dimethyl-1-(1,2,4-triazol-1-yl)-butanon in 30 ml Tetrahydrofuran. Man läßt das Reaktionsgemisch 15 Stunden bei Raumtemperatur und 4 Stunden unter Rückfluß rühren, kühlt ab und gibt auf Wasser. Man extrahiert mit Methylenchlorid, trocknet die organische Phase über Natriumsulfat und engt im Vakuum ein. Man erhält 15,4 g (73,7 % der Theorie) 2-(2,4-Dichlorphenyl-tert.-butyl)-2-(1,2,4-triazol-1-yl-methyl)-oxiran vom Brechungsindex $n_D^{20} = 1{,}5539$.

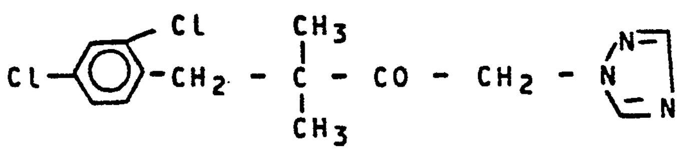

30 g (0,09 Mol) 1-Brom-4-(2,4-dichlorphenyl)3,3-dimethyl-2-butanon, 12,4 g (0,18 Mol) 1,2,4-Triazol und 24,8 g (0,18 Mol) Kaliumcarbonat werden in 300 ml Aceton 6 Stunden unter Rückfluß erhitzt. Danach läßt man abkühlen, saugt ab und engt die Mutterlauge im Vakuum ein. Der Rückstand wird in Methylenchlorid aufgenommen, mit Wasser gewaschen, über Natriumsulfat getrocknet und im

Vakuum eingeengt. Der Rückstand wird aus Diethylether umkristallisiert. Man erhält 12,8 g (45,6 % der Theorie) 4-(2,4-Dichlorphenyl)-3,3-dimethyl-1-(1,2,4-triazol-1-yl)-2-butanon vom Schmelzpunkt 85°C.

In entsprechender Weise werden die nachfolgenden Verbindungen der allgemeinen Formel (Ie)

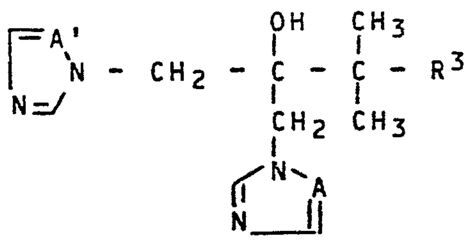

(Ie)

enthalten:

| Bsp.Nr. | $R^3$ | A | A' | Schmelzpunkt(°C) |
|---|---|---|---|---|
| Ie-2 | 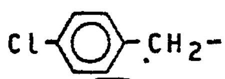 | N | N | 132 |
| Ie-3 | 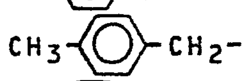 | N | N | 124 |
| Ie-4 | 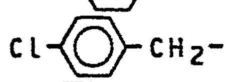 | N | CH | >220(xHCl) |
| Ie-5 | 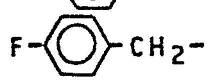 | N | N | 129 |

Es ist zwar bereits bekannt geworden, daß bestimmte Benzimidazol-Derivate, wie insbesondere 2-(α-Hydroxy-benzyl)-benzimidazol, in der Zellkultur antivirale Eigenschaften aufweisen. Am Versuchstier sind diese Verbindungen jedoch nur schwach wirksam bzw. unwirksam (vgl. Chemotherapy of Virus Diseases, Vol. I., Seite 115 bis 179, 1972). Außerdem ist die Wirkung dieser Verbindungen nur gegen Picorna-Viren gerichtet. Die Substanzen haben deshalb als antivirales Mittel in der Pharmazie keine Bedeutung erlangt. Demgegenüber weisen die erfindungsgemäßen Verbindungen eine ausgezeichnete antivirale Wirkung auf, wie die nachstehenden Versuchsergebnisse zeigen.

Beispiel A

Zellkultur-Versuch / Plaques-Reduktionstest

Der Plaques-Reduktionstest wurde nach der Methode von Herrmann et al. (Proc. Soc. Exp. Biol. Med. 103, 625-628 (1960) durchgeführt. L-929-Zellen der Maus wurden in Petrischalen ausgesät.

Jede Petrischlae enthält zwischen 1 bis 2,5 x $10^6$ Zellen. Zur Infektion wurde das Kulturmedium entfernt. Die Infektion der Zellen erfolgte mit 1 ml einer Virussuspension, die 5 x $10^3$ plaquebildende Einheiten und die Prüfsubstanz in einer nicht cytotoxischen Konzentration enthält.

Nach einer Stunde Adsorptionszeit wurde die Viruslösung entfernt und 5 ml an Überschichtungsmedium mit Prüfsubstanz zu jeder Petrischale gegeben.

Nach 3 - 4 Tagen wurden der Zellrasen angefärbt und die Virusplaques ausgezählt.

Die Anzahl von Plaques, die sich nach Infektion von Zellen mit Herpes simplex Virus ausbilden, sind bei infizierten und mit erfindungsgemäßen Verbindungen behandelten Zellen im Vergleich zu infizierten unbehandelten Zellen reduziert (vgl. Tabelle A).

T a b e l l e A:

Zellkultur-Versuch / Behandlung von L-929-Zellen, die mit Herpes simplex-Virus infiziert worden sind.

| Verbindung gemäß Herst. Beispiel Nr. | Antivirale Wirkung in Zellkultur |
|---|---|
| Ia-1 | + |
| Ia-3 | ++ |
| Ia-4 | ++ |
| Ic-1 | + |
| Ic-2 | + |
| Id-1 | + |
| Id-2 | + |
| Ie-2 | + |

++ = % Plaquesreduktion größer 50 %

\+ = % Plaquesreduktion kleiner 30 %

Beispiel B

Tierversuch / Kutan-Test am Meerschweinchen

Der Test wurde in Anlehnung an die von Huber et al (J.Invest. Dermatol. 62, 92-95, (1974)) ausgearbeitete Methode durchgeführt. 500 bis 500 g schwere Meerschweinchen wurden auf der Bauchseite enthaart und mit Nembutal (15 mg/kg i.p.) narkotisiert. Vorher markierte Hautareale wurden mit einer multiplen Impflanzette ('Vaccination Gun') infiziert. Als Virusmaterial wurde Medium von Kaninchennierenzellen benutzt, die mit Herpes simplex-Virus Typ I infiziert worden sind. Die Behandlung kann lokal, parenteral, oral, intraperitoneal oder intravenös erfolgen. Als Kontrolle dienten infizierte, nicht behandelte oder mit Placebo behandelte Tiere. Die Bewertung erfolgte nach Zahl und Größe der Herpesbläschen. Die Ergebnisse sind in der nachfolgenden Tabelle zusammengefaßt.

T a b e l l e   B

Tierversuch / Kutan-Test am Meerschweinchen

| Verbind. gemäß Herst. Bsp. | Antivirale Wirkungim Kutan-Test (Meerschweinchen) p.o.Behandlung | lokale Behandlung |
|---|---|---|
| Ia-2 | + | ++ |
| Ia-3 | ++ | ++ |
| Ia-4 | ++ | ++ |
| Ib-1 | + | nicht geprüft |
| Ib-2 | + | nicht geprüft |
| Id-2 | + | nicht geprüft |

++ = 50 % Reduktion der Zahl und Größe der Herpesbläschen

+ = 50 % Reduktion der Zahl und Größe der Herpesbläschen

Patentansprüche

1. Hydroxyethyl-azolyl-derivate der Formel

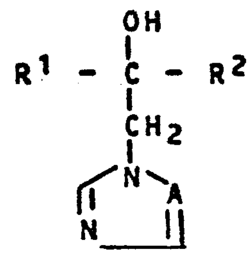

(I)

in welcher

A für ein Stickstoffatom oder die CH-Gruppe steht,

$R^1$ für jeweils gegebenenfalls im Phenylteil substituiertes Phenyl, Phenoxymethyl, Phenethyl oder Phenethenyl steht, sowie für Naphthyloxymethyl, 1,2,4-Triazol-1-yl-methyl oder Imidazol-1-yl-methyl steht,

$R^2$ für die Gruppierung $-C(CH_3)_2-R^3$, 1,2,4-Triazol-1-yl-methyl oder Imidazol-1-yl-methyl steht und

$R^3$ für Methyl sowie jeweils gegebenenfalls substituiertes Phenoxy oder Benzyl steht,

mit der Maßgabe, daß nicht gleichzeitig A für ein Stickstoffatom, $R^1$ für p-Chlorphenoxymethyl und $R^2$ für tert.-Butyl stehen,

und deren physiologisch verträglichen Säureadditions-Salze zur Verwendung bei der Bekämpfung von Virusinfektionen.

2. Hydroxyethyl-azolyl-derivate der Formel (I) in Anspruch 1, in welcher A die in Anspruch 1 angegebene Bedeutung hat und

$R^1$ für jeweils einfach bis dreifach, gleich oder verschieden im Phenylteil substituiertes Phenyl, Phenoxymethyl, Phenethyl oder Phenethenyl steht, wobei als Substituenten genannt seien: Halogen, Alkyl mit 1 bis 4 Kohlenstoffatomen, Alkoxy und Alkylthio mit jeweils 1 bis 2 Kohlenstoffatomen, Halogenalkyl und Halogenalkoxy sowei Halogenalkylthio mit jeweils 1 bis 2 Kohlenstoffatomen und 1 bis 5 gleichen oder verschiedenen Halogenatomen, wie Fluor- und Chloratomen, Phenyl und Halogenphenyl; ferner für Naphthyloxymethyl, 1,2,4-Triazol-1-yl-methyl und Imidazol-1-yl-methyl,

$R^2$ für die Gruppierung $-C(CH_3)_2-R^3$, für 1,2,4-Triazol-1-yl-methyl oder Imidazol-1-yl-methyl steht und

$R^3$ für Methyl sowie für jeweils gegebenenfalls einfach bis dreifach, gleich oder verschieden substituiertes Phenoxy oder Benzyl steht, wobei als Substituenten vorzugsweise die bei $R^1$ bereits vorzugsweise genannten Phenylsubstituenten in Frage kommen, mit der Maßgabe, daß nicht gleichzeitig A für ein Stickstoffatom, $R^1$ für p-Chlorphenoxymethyl und $R^2$ für tert.-Butyl stehen,

zur Verwendung bei der Bekämpfung von Viruserkrankungen.

3. Verwendung von Hydroxyethyl-azolyl-Derivaten der Formel (I)

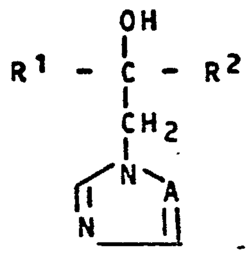

(I)

in welcher

A für ein Stickstoffatom oder die CH-Gruppe steht,

$R^1$ für jeweils gegebenenfalls im Phenylteil substituiertes Phenyl, Phenoxymethyl, Phenethyl oder Phenethenyl steht, sowie für Naphthyloxymethyl, 1,2,4-Triazol-1-yl-methyl oder Imidazol-1-yl-methyl steht,

$R^2$ für die Gruppierung $-C(CH_3)_2-R^3$, 1,2,4-Triazol-1-yl-methyl oder Imidazol-1-yl-methyl steht und

$R^3$ für Methyl sowie jeweils gegebenenfalls substituiertes Phenoxy oder Benzyl steht,

mit der Maßgabe, daß nicht gleichzeitig A für ein Stickstoffatom, $R^1$ für p-Chlorphenoxymethyl und $R^2$ für tert.-Butyl stehen,

und/oder deren physiologisch verträglichen Säureadditions-Salzen zur Bekämpfung von Viruserkrankungen.

4. Verwendung von Hydroxyethyl-azol-Derivaten der Formel (I) in Anspruch 3, in welcher A die in Anspruch 3 angegebene Bedeutung hat und

$R^1$ für jeweils gegebenenfalls einfach bis dreifach, gleich oder verschieden im Phenylteil substituiertes Phenyl, Phenoxymethyl, Phenethyl oder Phenethenyl steht, wobei als Substituenten genannt seien: Halogen, Alkyl mit 1 bis 4 Kohlenstoffatomen, Alkoxy und Alkylthio mit jeweils 1 bis 2 Kohlenstoffatomen, Halogenalkyl und Halogenalkoxy sowie Halogenalkylthio mit jeweils 1 bis 2 Kohlenstoffatomen und 1 bis 5 gleichen oder verschiedenen Halogenatomen, wie Fluor- und Chloratomen, Phenyl und Halogenphenyl; ferner für Naphthyloxymethyl, 1,2,4-Triazol-1-yl-methyl und Imidazol-1-yl-methyl;

$R^2$ für die Gruppierung $-C(CH_3)_2-R^3$, für 1,2,4-Triazol-1-yl-methyl oder Imidazol-1-yl-methyl steht; und

$R^3$ für Methyl sowie für jeweils gegebenenfalls einfach bis dreifach, gleich oder verschieden substituiertes Phenoxy oder Benzyl steht, wobei als Substituenten vorzugsweise die bei $R^1$ bereits vorzugsweise genannten Phenylsubstituenten in Frage kommen; mit der Maßgabe, daß nicht gleichzeitig A für ein Stickstoffatom, $R^1$ für p-Chlorphenoxymethyl und $R^2$ für tert.-Butyl stehen,

zur Bekämpfung von Viruserkrankungen.

5. Verwendung von 1-(4-Chlorphenyl)-4,4-dimethyl-3-(1,2,4-triazol-1-yl-methyl)-pentan-3-ol bei der Bekämpfung von Viruserkrankungen.

6. Verwendung von 1-(4-Phenylphenyl)-oximethyl-3,3-dimethyl-2-(imidazol-1-yl)-butan-2-ol bei der Bekämpfung von Viruserkrankungen.

7. Verwendung von 1-(4-Phenylphenyl)-oximethyl-3,3-dimethyl-2-(imidazol-1-yl)-butan-2-ol bei der Bekämpfung von Viruserkrankungen.

8. Antivirales Mittel, gekennzeichnet durch einen Gehalt an mindestens einem Hydroxyethyl-azolyl-Derivat der Formel (I)

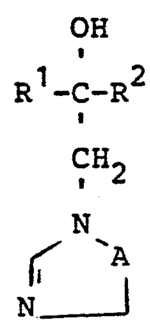

(I)

in welcher

A für ein Stickstoffatom oder die CH-Gruppe steht,

$R^1$ für jeweils gegebenenfalls im Phenylteil substituiertes Phenyl, Phenoxymethyl, Phenethyl oder Phenethenyl steht, sowie für Naphthyloxymethyl, 1,2,4-Triazol-1-yl-methyl oder Imidazol-1-yl-methyl steht,

$R^2$ für die Gruppierung $-C(CH_3)_2-R^3$, 1,2,4-Triazol-1-yl-methyl oder Imidazol-1-yl-methyl steht und

$R^3$ für Methyl sowie jeweils gegebenenfalls substituiertes Phenoxy oder Benzyl steht,

mit der Maßgabe, daß nicht gleichzeitig A für ein Stickstoffatom, $R^1$ für p-Chlorphenoxymethyl und $R^2$ für tert.-Butyl stehen,

und/oder deren physiologisch verträglichen Säureadditions-Salzen.

9. Verfahren zur Herstellung eines antiviralen Mittels, gemäß Anspruch 12, dadurch gekennzeichnet, daß man Hydroxyethyl-azolyl-Derivate gemäß der Formel (I) in Anspruch 8 mit inerten, nichttoxischen, pharmazeutisch geeigneten Trägerstoffen vermischt.